# EUROPÄISCHE PATENTSCHRIFT

(11) **EP 0 499 055 B1**
(45) Veröffentlichungstag und Bekanntmachung des Hinweises auf die Patenterteilung: **22.04.1998**
(21) Anmeldenummer: 92100959.3
(22) Anmeldetag: 22.01.1992
(51) Int. Cl.: C07C 43/23, C07C 39/08, C07C 41/28, C07C 37/50

(54) **Verfahren zur Herstellung von Brenzcatechinen**
Process for the preparation of pyrocatechols
Procédé pour la préparation de pyrocatéchines

(30) Priorität: 14.02.1991 DE 4104417
(43) Veröffentlichungstag der Anmeldung: 19.08.1992
(73) Patentinhaber: BASF Aktiengesellschaft, 67063 Ludwigshafen (DE)
(72) Erfinder: Karcher, Michael, Dr., W-6915 Dossenheim (DE); Zimmermann, Horst, Dr., W-6800 Mannheim 24 (DE); Henkelmann, Jochem, Dr., W-6700 Ludwigshafen (DE)

(56) Entgegenhaltungen:
- DE-A- 2 064 097
- DE-A- 2 130 392
- GB-A- 2 051 068
- CHEMICAL ABSTRACTS, vol. 82, no. 23, 9. Juni 1975, Columbus, Ohio, US; abstract no. 155783z, G. SUGERMAN 'CATALYTIC METHOD FOR MAKING CATECHOL AND ALKOXY DERIVATIVES' Seite 558 ;Spalte 1 ;

## Beschreibung

Die vorliegende Erfindung betrifft ein neues Verfahren zur Herstellung von Brenzcatechinen der allgemeinen Formel I 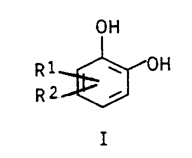 in der R¹ und R² für Wasserstoff oder C₁-C₈-Kohlenwasserstoffreste stehen

Die Verbindungen I sind bekannt und dienen als Zwischenprodukte für die Synthese von Pharmaprodukten, Riech- und Geschmacksstoffen.

In Ind. Eng. Chem., Prod. Res. Dev., 15 (3), 212 (1976) ist die katalytische Hydroxylierung von Phenol mit Wasserstoffperoxid in Gegenwart von Phosphorsäure und Perchlorsäure zu Brenzcatechin und Hydrochinon beschrieben.

Dieses Syntheseverfahren hat jedoch den Nachteil, daß man neben dem Brenzcatechin noch etwa gleiche Mengen Hydrochinon erhält.

Die DE-A 20 64 097 lehrt die Herstellung von Brenzcatechinen und deren Monoethern durch Umsetzung von 2-Alkoxycyclohexanonen oder Cyclohexandion-(1,2)-monoenolethern in der Gasphase an Palladium auf Li-Al-Spinell-Trägern sowie die Umsetzung von 2,2-Dimethoxycyclohexanon zum Brenzcatechinmonoether Guajakol.

Die GB-A 2 051 068 betrifft die Dehydrierung von 2-Alkoxycyclohexanolen zu Brenzcatechinmonoethern in flüssiger Phase oder in der Gasphase an einem Palladiumkatalysator.

Chem. Abst. 82, Nr. 23,155783 Z beschreibt die Umsetzung von 1,2-Cyclohexandionen und 2-Hydroxy-oder 2-Alkoxcyclohexanonen in flüssiger Phase an Edelmetallkatalysatoren oder in der Gasphase an Cu-, Cr- oder Ni-Katalysatoren.

Aus der DE-A 20 07 737 ist die partielle Veretherung von Brenzcatechin mit Alkylhalogeniden in schwach wäßrig-alkalischem Medium zu Brenzcatechinmonoalkylethern bekannt.

Eine weitere Synthesemöglichkeit der Brenzcatechinmonomethylether ist die partielle Methylierung des Brenzcatechins mit Methanol bei höheren Temperaturen in Gegenwart von Katalysatoren wie Phosphorsäure (DE-A 12 25 665) oder Phosphaten wie Borphosphat (DE-A 21 40 738).

Diese bisher bekannten Verfahren zur Herstellung der Brenzcatechinmonoalkylether vermögen jedoch sowohl aufgrund der, insbesondere bei hohen Umsätzen, vermehrt auftretenden Bildung von Dialkylethern und der damit verbundenen Trennprobleme als auch des hohen Verbrauchs an Alkylierungsmitteln nicht zu befriedigen.

Ferner ist es aus der DE-A-27 03 077 bekannt, Cyclohexandion-(1,4)-tetramethyldiketal durch katalytische Dehydrierung und Methanolabspaltung in der Flüssigphase zum Hydrochinondimethylether umzusetzen.

Der Erfindung lag die Aufgabe zugrunde, Brenzcatechine I auf einfachere und wirtschaftlichere Weise zugänglich zu machen.

Demgemäß wurde ein neues Verfahren zur Herstellung von Brenzcatechinen der allgemeinen Formel I 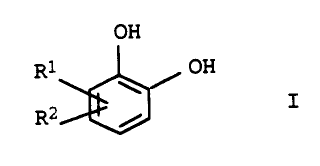 in der R¹ und R² für Wasserstoff oder C₁-C₈-Kohlenwasserstoffreste stehen, gefunden, welches dadurch gekennzeichnet ist, daß man ein 2-Hydroxycyclohexanondialkylketal der allgemeinen Formel II 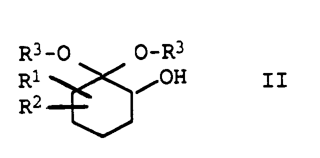 in der R³ einen C₁-C₈-Kohlenwasserstoffrest bedeutet, in Gegenwart eines Platinmetalls oder einer Verbindung eines dieser Metalle als Katalysatoren in der Gasphase und in Gegenwart von Wasser umsetzt.

Das erfindungsgemäße Verfahren läßt sich wie folgt veranschaulichen:

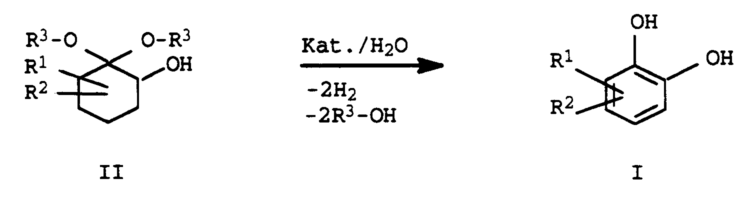

Die Ausgangsverbindungen II sind bekannt oder noch bekannten Methoden erhältlich, z.B. durch elektrochemische Oxidation von Cyclohexanon mit Alkanolen in Gegenwart eines Hilfselektrolyten und Wasser (DE-A 40 17 576).

Unter den definitionsgemäßen Verbindungen II werden aus wirtschaftlichen Gründen solche bevorzugt, in denen die Reste folgende Bedeutung haben:
- Wasserstoff;
- C₁-C₈-Alkyl, vorzugsweise C₁-C₄-Alkyl, darunter Vorzugsweise C₁-C₂-Alkyl wie Ethyl und vor allem Methyl;
- C₂-C₈-Alkenyl, Vorzugsweise C₂-C₆-Alkenyl, darunter vorzugsweise C₂-C₄-Alkenyl wie Propenyl, Butenyl und vor allem Ethenyl;
- C₂-C₈-Alkinyl, vorzugsweise C₂-C₆-Alkinyl, darunter vorzugsweise C₂-C₄-Alkinyl wie Propinyl, Butinyl und vor allem Ethinyl;
- C₃-C₈-Cycloalkyl, vorzugsweise C₅-C₇-Cycloalkyl wie Cyclopentyl, Cycloheptyl und besonders Cyclohexyl;
- C₇-C₈-Arylalkyl, vorzugsweise Phenylethyl und ganz besonders Benzyl;
- Aryl wie vorzugsweise Phenyl.

Im Hinblick auf die gewünschten Verfahrensprodukte I ist das 2-Hydroxycyclohexanondimethylketal besonders bevorzugt.

Aus verfahrenstechnischen Gründen empfiehlt es sich, die 2-Hydroxycyclohexanondimethylketale II in Form von Lösungen einzusetzen und zusammen mit dem Lösungsmittel zu verdampfen.

Zur Gewinnung der Verbindungen I ist Wasser, auch in Form von Gemischen, als Lösungsmittel bevorzugt.

Im allgemeinen enthalten die Lösungen 10 bis 80 Gew.-%, bevorzugt 20 bis 50 Gew.-% der Verbindung II.

Die im erfindungsgemäßen Verfahren einzusetzenden Katalysatoren sind Platinmetalle oder Verbindungen eines dieser Metalle.

Man kann die Katalysatoren in metallischer Form, in Form von Salzen und Oxiden oder in Form von Komplexverbindungen einsetzen.

Als Katalysatoren in metallischer Form eignen sich bevorzugt Platin und insbesondere Palladium.

Vorzugsweise verwendet man die Metalle in Form von Trägerkatalysatoren auf Trägern mit möglichst großer Oberfläche. Bevorzugte Träger sind Aluminiumoxid, Siliciumoxid, Titanoxid, Zinkoxid, Lanthanoxid, Zirkonoxid, Bariumsulfat, Aluminiumsilikat, Spinelle oder Gemische dieser Materialien sowie vor allem Kohle, wobei Palladium auf Kohle besonders hervorzuheben ist.

Die Trägerkatalysatoren haben vorzugsweise einen Gehalt an aktiven Metallen von 0,2 bis 5, besonders 0,6 bis 1,5 Gew.-%, bezogen auf die Summe aus Träger und katalytisch aktiven Metallen, berechnet als Metall.

Die Katalysatoren können wahlweise als 2- bis 4-mm-Stränge oder als Tabletten mit 3 bis 5 mm Durchmesser, als Splitt mit Teilchengrößen von 0,05 bis 1 mm, vorzugsweise 0,1 bis 0,5 mm als größten Durchmesser oder als Pulver mit Teilchengrößen von 0,05 bis 0,5 mm eingesetzt werden, wobei sich das Pulver auch als Wirbelkontakt eignet.

Verwendet man die definitionsgemäßen Katalysatormetalle in Form ihrer Verbindungen, so eignen sich hierbei besonders Komplexverbindungen des Rutheniums und Rhodiums, beispielsweise Rhodiumacetylacetonat sowie Komplexe mit mindestens zwei Liganden vom Typ der Triorganylphosphorverbindungen und Kohlenmonoxid als weitere Liganden.

Die Katalysatorbelastung beträgt beim kontinuierlichen Verfahren 0,1 bis 20 kg, vorzugsweise 1 bis 5 kg der Verbindung II pro kg des Metalls.

Wirtschaftlich sehr vorteilhaft ist es, die Platinmetallkatalysatoren in partiell desaktivierter Form einzusetzen, da dies, insbesondere bei hochaktiven Metallen, zu höherer Produktselektivität führt.

Als desaktivierende Katalysatorgifte eignen sich bevorzugt Schwefel, Selen, Tellur, Jod und Bariumsulfat, die im allgemeinen in Mengen von 0,05 bis 0,3 Gew.-%, Vorzugsweise 0,1 bis 0,15 Gew.-% der Verbindung II eingesetzt werden.

Üblicherweise führt man die Reaktion bei 200 bis 400°C, vorzugsweise bei 240 bis 340°C und ganz besonders bevorzugt bei 280 bis 300°C durch. Da in der Gasphase umgesetzt wird, muß die Temperatur mindestens so hoch gewählt werden, daß ein vollständiges Verdampfen der Ausgangsverbindung II gewährleistet ist.

Zweckmäßigerweise wird die Umsetzung bei Normaldruck vorgenommen, jedoch kann man auch bei vermindertem oder leicht erhöhtem Druck arbeiten, also etwa im Bereich von 10 mbar bis 20 bar.

Die Reaktionszeiten betragen normalerweise 0,1 bis 60 Sekunden, meistens 1 bis 5 Sekunden.

Es kann zweckmäßig sein, zur Unterdrückung von Dehydratisierungsprozessen die Umsetzung unter Mitverwendung von basischen Zusätzen durchzuführen. Hierbei eignen sich Stickstoffbasen wie Triethylamin, Cyclohexylamin und Pyridin. Bevorzugt sind Alkalihydroxide wie Natron- und Kalilauge und Alkalialkoholate wie Natriummethanolat, Kaliummethanolat und Natriumethanolat. Besonders bevorzugte Basen sind Alkalicarbonate wie Natriumcarbonat und vor allem Kaliumcarbonat.

Diese werden üblicherweise in Mengen von 0,1 bis 3,0 Gew.-%, vorzugsweise von 1,0 bis 1,5 Gew.-% der Verbindungen II eingesetzt.

Die Umsetzung erfolgt in der Gasphase, wobei eine Festbettreaktion und eine Gasphasenreaktion im Wirbelbett in Betracht kommen.

Um die Katalysatoraktivität während der Dehydrierung aufrechtzuerhalten, empfiehlt es sich, ein Trägergas aus Wasserstoff oder einem Gemisch aus Wasserstoff und Stickstoff oder Argon zu verwenden.

Eine wirtschaftlich sehr vorteilhafte Ausführungsform des erfindungsgemäßen Verfahrens besteht in der Festbettreaktion, indem man das 2-Hydroxycyclohexanondialkylketal II, gegebenenfalls in Lösung, verdampft und als Gasphase, gegebenenfalls mit Hilfe eines Trägergases, über einen festen Katalysator leitet.

Für den Fall der lösungsmittelfreien Zugabe der Verbindung II wird zur Gewinnung der Verfahrensprodukte I Wasser in Form von Wasserdampf zugesetzt.

Man kann die Aufarbeitung des Gemisches auf die Verfahrensprodukte in an sich bekannter Weise vornehmen, und zwar in der Regel durch fraktionierende Destillation des Reaktionsgemisches und gegebenenfalls anschließende Extraktion.

Die nach dem erfindungsgemäßen Verfahren auf einfache und wirtschaftliche Weise erhältlichen Brenzcatechine I können als Zwischenprodukte zur Synthese von Pharmaprodukten wie Verapamil, von Riechstoffen und Geschmacksstoffen wie Vanillin verwendet werden.

### Beispiel

### Herstellung von Brenzcatechin

Innerhalb einer Stunde wurde eine Lösung aus 6 g 2-Hydroxycyclohexanondimethylketal in 24 g Wasser bei einer Temperatur von 300°C verdampft und wurde zusammen mit 60 l eines 1:1-Gemisches aus Wasserstoff und Stickstoff als Trägergas mit einer Verweilzeit von 2 Sekunden über 100 g eines festen Palladiumkatalysators (1 Gew.-% Palladium auf Kohle; vergiftet mit 0,1 Gew.-% Schwefel; basischer Zusatz : 1,5 Gew.-% Kaliumcarbonat) geleitet.

Die übliche Aufarbeitung durch fraktionierende Destillation führte zu einer Ausbeute an Brenzcatechin von 74,1 %.

Bei Verwendung einer 9 : 1-Mischung aus Methanol und Wasser erhielt man eine Produktmischung aus 30,70 % Brenzcatechinmonomethylether und 23,3 % Brenzcatechin.

## Patentansprüche

1. Verfahren zur Herstellung von Brenzcatechinen der allgemeinen Formel 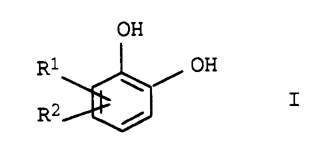 in der R¹ und R² für Wasserstoff oder C₁-C₈-Kohlenwasserstoffreste stehen, dadurch gekennzeichnet, daß man ein 2-Hydroxycyclohexanondialkylketal der allgemeinen Formel II 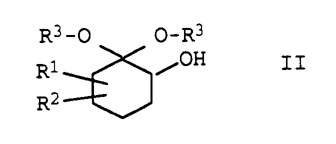 in der R³ einen C₁-C₈-Kohlenwasserstoffrest bedeutet, in Gegenwart eines Platinmetalls oder einer Verbindung eines dieser Metalle als Katalysatoren in der Gasphase und in Gegenwart von Wasser umsetzt.

2. Verfahren nach Anspruch 1, dadurch gekennzeichnet, daß die Reste R¹ und R² für Wasserstoff stehen und R³ eine Methylgruppe bedeutet.

3. Verfahren nach den Ansprüchen 1 oder 2, dadurch gekennzeichnet, daß man partiell desaktiverte Platinmetalle als Katalysatoren einsetzt.

## Claims

1. A process for the preparation of pyrocatechols of the formula 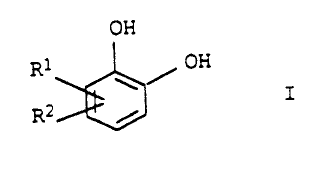 where R¹ and R² are hydrogen or C₁-C₈-hydrocarbon radicals, which comprises reacting a 2-hydroxycyclohexanone dialkyl ketal of the formula II 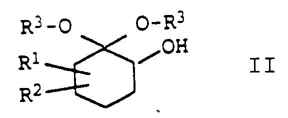 where R³ is a C₁-C₈-hydrocarbon radical, in the gas phase in the presence of a platinum metal or of a compound of one of these metals as catalyst and in the presence of water.

2. A process as claimed in claim 1, wherein R¹ and R² are hydrogen and R³ is methyl.

3. A process as claimed in claim 1 or 2, wherein the catalyst employed is a partially deactivated platinum metal.

## Revendications

1. Procédé de préparation de benzocatéchines de formule générale 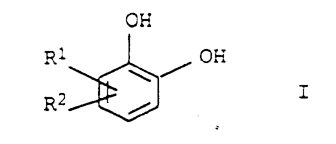 dans laquelle R¹ et R² sont mis pour des atomes d'hydrogène ou des restes hydrocarbonés en C₁-C₈, caractérisé en ce qu'on met en réaction un 2-hydroxycyclohexanonedialkylcétal de formule générale II 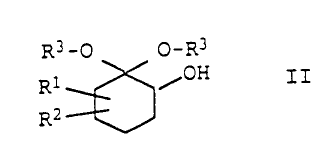 dans laquelle R¹ rerésente un reste hydrocarboné en C₁-C₈, en présence d'un métal du groupe du platine ou d'un composé d'un de ces métaux comme catalyseur, en phase gazeuse et en présence d'eau.

2. Procédé selon la revendication 1, caractérisé en ce que les restes R¹ et R² sont mis pour un atome d'hydrogène et R³ pour un groupement méthyle.

3. Procédé selon l'une ou l'autre des revendications 1 et 2, caractérisé en ce qu'on utilise comme catalyseurs des métaux du groupe du platine partiellement désactivés.
